# EUROPEAN PATENT APPLICATION

(11) **EP 4 706 606 A1**
(43) Date of publication of application: **11.03.2026**
(21) Application number: 25170029.0
(22) Date of filing: 11.04.2025
(51) Int. Cl.: A61F 9/007

(54) **PNEUMATIC SPRING-LOADED VITREORETINAL BRUSH**

(30) Priority: 05.09.2024 IT 202400012664
(71) Applicant: Idia Societá A Responsabilitá Limitata Semplificata, 95049 Vizzini Catania (IT)
(72) Inventor: BRANCATO, CLAUDIO, 90151 PALERMO (IT); LO GIUDICE, GREGORIO, 95049 VIZZINI (IT)
(74) Representative: Randazzo, Luigi

(57) **Abstract**

The present invention relates to the field of automated ophthalmic surgical instruments, used for the surgical treatment of vitreoretinal diseases. The invention refers, in particular, to an automatic vitreous brush suitable for removing the vitreous body and, in particular, the posterior hyaloid adhered to the retina, comprising an elongated tubular element 2, an oblong head 3 equipped with a rack system 4, a set of bristles 5, an air compressor 6, a detection system, characterised by a movement member suitable for moving said oblong head 3, in which said set of bristles 5 is located, which, by defining a rotary motion of said oblong head 5, ensures the removal of the vitreous body while keeping the retinal tissue intact and, therefore, the absence of intraoperative complications.

## Description

The present invention relates to ophthalmic surgical instruments and in particular to those used for the removal of the vitreous body and of the posterior hyaloid applied to the retina, for the treatment of vitreous-retinal pathologies.

### - Description of the prior art (background)

It Is commonly known that retinal detachment occurs due to retinal breakages caused by retinal tremors and/or vitreous adhesions. In particular, the main cause of retinal detachment is the insinuation of a certain quantity of vitreous fluid below the retina, which begins to detach from the underlying tissues.

The vitreous base is a sort of gelatin mainly formed by water, but also by collagen fibers which give the consistency of the gel.

The vitreous body is contained by a thin membrane, the hyaloid, and is adhered to the retina with points of union exclusively around the optical papilla and the vitreous base near the clamped hour.

The objective of ophthalmic surgery for the treatment of retinal detachment is the integral elimination of the vitreous fluid inside the eyeball and the ability to "brush" the vitreous fluid from the retina; the smaller the amount of vitreous fluid adhering to the retina, the greater the chances of retinal success. In fact, incomplete removal of vitreous or formation of iatrogenic retinal holes causes formation of vitreous retinal proliferations, causing retinal detachment.

At present, the separation of the vitreous, and in particular of the posterior hyaloid, from the retina is carried out by means of mechanically actuated instruments, such as aspiration apparatuses, cannulas, buffers and the like.

In particular, the basic instrument used in the above mentioned ophthalmic interventions is vitrectomo, or a pneumatic guillotine device suitable for cutting the vitreous gel and sucking *the* vitreous humor present inside the eyeball.

In some vitreous conditions, the separation of the vitreous from the retina, by means of non-automated manual devices, is a difficult and problematic operation, since the latter are not able to remove the vitreous adhesions in a precise and controlled manner. In fact, the manual devices currently in use require a very high experience and precision of the operator during the execution of the intervention, since it is very complex to control and modulate the suction speed and, therefore, to carry out exactly the total removal of the vitreous body and of the posterior hyaloid applied to the retina.

This results in the occurrence of a series of intraoperative and post-operative complications, such as vitreous hemorrhages, continuous solutions in retinal and coroideal tissue, etcc retinal lifts.

Another problem related to the prior art is represented by the tensile forces exerted on the retina. Specifically, at the peripheral retinal region, the vitreous is significantly adhered to the retina itself and, therefore, when the operator removes the vitreous in this specific region, the retina is subjected to variable tensile forces which can cause damage or even detachment thereof.

In this scenario, the technical task of the present invention is to standardize the surgical procedure for removal of the posterior hyaloid and, therefore, to avoid the onset of complications (for example, vitreous-retinal hemorrhages, continuous solutions in retinal and coroideal tissue, retinal lifts, etcc), related to the use of the manual devices existing in the state of the art.

This object is achieved by the present invention, as claimed in the appended claims. It consists of an automated vitreous toothbrush with assisted operation by a vitrectomy device suitable for the removal of the vitreous body, and in particular of the posterior hyaloid applied to the retina.

The present invention is composed of an elongated tubular element, an oblong head, in which there is a bristle assembly, made of material such as nylon 10/0, provided with a rack system. designed to easily carry out movements on the vitreous body and to effectively and safely clean the vitreous adhesions.

This object is achieved thanks to the automated nature of the invention, which allows to achieve constant and defined movements during the separation and cleaning process.

With respect to existing manual techniques, which are unable to guarantee precise movements and ensure a constant pressure, the automatic toothbrush follows an automated procedure preset by the operator, greatly reducing the likelihood of causing injury, trauma and/or damage to delicate retinal tissues.

Moreover, the oblong head allows greater coverage and accessibility to the vitreous adhesions on the retina and this results in a complete and effective cleaning. Moreover, the rack system ensures a regular and controlled movement of the head, considerably simplifying the removal of the adherences, avoiding damage and/or injury to the surrounding retinal tissue.

Another advantage of the invention also lies in the functionality combined with a vitrectomy device. In fact, the simultaneous action of the two devices allows the operator to monitor and modulate the speed, pressure and all the other parameters of the toothbrush, in real time and in relation to the specific eye conditions of each patient.

The *design* of the toothbrush, the materials and coatings of which it is composed are also functional to considerably reduce the risk of contamination of foreign particles inside the eye and/or possible damage to the tissues.

The invention is also provided with a pneumatic system consisting of a compressor to ensure a constant and controlled air flow. The release of the air jet guaranteed by the compressor ensures an efficient cleaning of the vitreous adherences on the retina, allowing to keep the retina or the macula attached and functioning.

The above objects are achieved by means of the present invention, in accordance with claim 1, consisting of:
- A tubular handle from which an automatic actuation system of the vitreous brush is actuated;
- an oblong head composed of bristles made of material such as Nylon 10/0, equipped with a rack mechanism, driven by an automatic system;
- an air compressor to ensure a constant and controlled air flow.

The structure of the invention is provided to be compatible with vitreous retinal systems of different gauges, such as 23/25/27 gauge, offering flexibility and adaptability to the various surgical instruments and procedures.

The device, thus designed, can be used both for vitrectomy operations but also in vitreous-retinal surgical procedures of regmatogenic retinal detachment with or without vitreous-retinal proliferation (PVR), macular hole , macular pucker, proliferative diabetic retinopathy with or without associated haemovitreo and/or retinal detachment and other retinal vitreous pathologies of surgical relevance.

### ·Brief description of the drawings

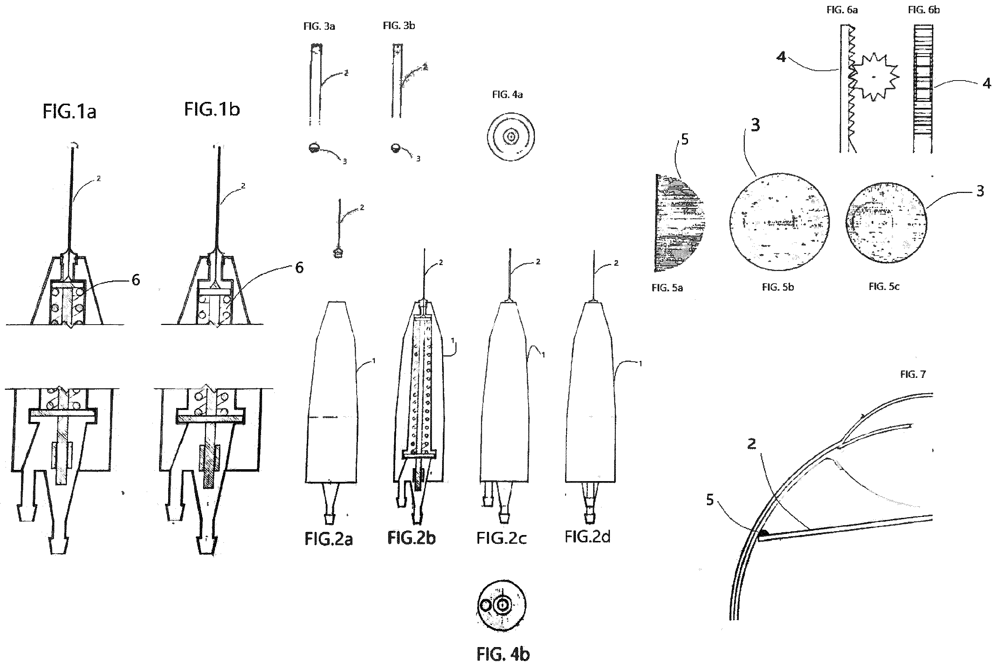

the features and advantages of the invention will become more apparent in the light of the detailed description of a preferred but not exclusive embodiment, illustrated by way of non-limiting example with reference to the accompanying drawings on a scale, in which the figures show the elements described below:
- figure la and 1b:
- show two sections of the device; - figure 2a: show the external body of the device with an exploded view of the head;
- figure 2b: shows the device in longitudinal section; - figure 2c -2d: figures
   3a and 3b show the detail of the elongated tubular element and the oblong head;
- figure 4a-4b shows the device in a top view; - figure
   5a: figure
   5b and 5c shows a front view of the gauge head 23 and gauge 25: - figure
   6a-6b: show two details of the rack system; - figure
   7: shows a cross-section of the eye, in which the device is shown inside the eyeball adjacent to the vitreous-retinal components.

### Description of at least one embodiment of the invention with reference to the drawings

the present invention will now be described by way of non-limiting example, according to a preferred embodiment, also referring to the figures described which show the components of the instrument.

In surgical operations of vitrectomy it is necessary to insert three trocar into the eye via trans-scleral . The device is inserted inside the eyeball using as access route a *trocar* assisted by an endo-illuminator (inserted through another *trocar).* The oblong head 3, in which the bristles 5 are placed at the end in the distal vicinity, is inserted inside the eyeball into the vitreous cavity through the trocar scleral, in a position adjacent to the retina and, therefore, to the vitreous components (for example, the posterior hyaloid and/or vitreous-retinal membranes of surgical relevance). The operator then operates the rack system 4. Once the brush head 3 has been actuated, the hyaloid is "swept" by means of the introduction of compressed air 6 with a circumferential or rotary mechanism.

The air compressor 6 functions as a pneumatic system and is activated by the air introduced into the handle and the spring system will be designed to slide the rack to which the bristles are attached at its end.

At this point the instrument can be deactivated and n extracted from the eye.

The object of the invention is susceptible of numerous modifications and variations of a practical and applicational nature, all of which are within the scope of the inventive concept set forth in the appended claims. All the details may be replaced with other technically equivalent elements and the materials may be different according to requirements, without departing from the scope of protection of the present invention.

Although the object has been described with particular reference to the accompanying drawings, the reference numerals used in the description and in the claims are used to improve the intelligence of the invention and do not constitute any limitation to the scope of protection claimed.

## Claims

1. Automatic device for ophthalmic surgery operations and in particular for the surgical treatment of vitreoretinal pathologies, suitable for removing the vitreous body and the posterior hyaloid attached to the retina, comprising a tubular handle (1) from which a plurality of mobile elements of the device, respectively:
- an elongated tubular element (2);
- an oblong head (3) equipped with a rack system (4);
- bristle complex (5);
- air compressor (6);
**characterized by** the fact that:
said device consists of a toothbrush in which said air compressor (6), through the introduction of air into the device, activates said oblong head (3), in which said set of bristles (5) insist, defining, by means of a spring system, a rotary motion of said oblong head (3), creating constant and defined movements through automatic operation.

2. Automatic device according to claim no. 1, wherein said oblong head (3) constitutes the end of the elongated tubular element (2).

3. Automatic device according to claim no. 1, wherein each bristle of said bristle complex (5) has a measurement equal to 20 microns.

4. Automatic device according to claim no. 1, in which said air compressor (6) creates a pneumatic system which bases its operation on the generation of compressed air produced through said air compressor (6), which by introducing air inside the device activates said head and through a spring system it acts on said rack system ensuring precise and defined cleaning of the vitreous adhesions without damaging the retinal tissue.

5. Automatic device according to claim no. 1, wherein said device comprises a detection and feedback system.

## Amended claims

### Amended claims in accordance with Rule 137(2) EPC.

1. Automatic device for ophthalmic surgery operations and in particular for the surgical treatment of vitreoretinal pathologies, for the removal of the vitreous body and the posterior hyaloid adhered to the retina, comprising:
- a main body (1) configured to be gripped by a hand;
- a guide tube (2) coupled to the end of the said main body (1);
- a lever installed on said main body (1);
- connection member inserted into the said guide tube (2) and connected to said lever;
- an oblong head (3) equipped with a rack system (4), adapted to be exposed outside the guide tube (2) or retracted inside said guide tube (2) by operation of the said lever, said oblong head (3) configured to separate the vitreous cortex adhered to the retina when exposed outside the said guide tube (2);
- set of bristle (5);
**characterized by** the fact that:
the device consists an automated vitreous cleaning head in the form of a rotary brush, actuated by an air compressor (6) configured to introduce air into the device to drive said oblong head (3),provided with said rack system (4) and said set of bristles (5) said air compressor (6) operating in combination with a spring mechanism to generate controlled and defined rotational movements of said oblong head (3), thereby ensuring safe and
effective cleaning of vitreoretinal adhesions while maintaining the retina or macula attached and functional.

2. Automatic device according to claim no. 1, wherein said oblong head (3) constitutes the end of the said connection member.

3. Automatic device according to claim 1, wherein each bristle of said set of bristle (5) has a size of 20 microns.

4. Automatic device according to claim 1, wherein said air compressor (6) creates a pneumatic system which bases its operation on the generation of compressed air produced through said air compressor (6), which by introducing air inside the device activates said oblong head (3) and by means of a spring mechanism acts on said rack system ensuring precise and defined cleaning of the vitreous adhesions without damaging the retinal tissue.

5. Automatic device according to claim 1, wherein said device comprises a detection and feedback system.
